# EUROPEAN PATENT APPLICATION

(11) **EP 4 779 650 A1**
(43) Date of publication of application: **22.07.2026**
(21) Application number: 25153165.3
(22) Date of filing: 21.01.2025
(51) Int. Cl.: G16H 50/20, A61B 5/01, A61B 5/00, G16H 50/30

(54) **A METHOD FOR IDENTIFYING AN INFECTION RISK AT A WOUND SITE**

(71) Applicant: Mölnlycke Health Care AB, 431 21 Mölndal (SE)
(72) Inventor: KIDBORG, Stefan, 442 54 YTTERBY (SE); SCHANTZ, Anna, 413 23 GÖTEBORG (SE)
(74) Representative: Kransell & Wennborg KB

(57) **Abstract**

The present disclosure relates to a computer-implemented method, system, and corresponding computer program product for identifying an infection risk at a wound site through continuous temperature monitoring. The disclosed solution involves acquiring temperature data from a plurality of sensors positioned near the wound site at predefined intervals, processing the data to form a time-series of temperature values, and analyzing the time-series to detect a temperature trend. Deviations from a predefined baseline temperature pattern associated with normal wound healing are identified, and an alert signal is generated upon detecting a deviation indicative of a potential infection risk.

## Description

### TECHNICAL FIELD

The present disclosure relates to a computer-implemented method, system, and corresponding computer program product for identifying an infection risk at a wound site through continuous temperature monitoring. The disclosed solution involves acquiring temperature data from a plurality of sensors positioned near the wound site at predefined intervals, processing the data to form a time-series of temperature values, and analyzing the time-series to detect a temperature trend. Deviations from a predefined baseline temperature pattern associated with normal wound healing are identified, and an alert signal is generated upon detecting a deviation indicative of a potential infection risk.

### BACKGROUND

In medical wound care, monitoring wound site temperature is essential for assessing healing progress and identifying potential complications. Elevated or irregular patient temperature patterns may indicate early signs of an infection, making temperature a parameter for continuous assessment. Effective temperature monitoring could offer healthcare professionals valuable, early-stage information to guide treatment decisions, including infection prevention measures, wound dressing changes, and tailored wound care interventions.

Traditionally, healthcare providers rely on manual inspection of wound sites to assess temperature-related signs of infection. This often involves physical checks or external thermometers and may require disturbing the wound dressing to access the wound area directly. Such manual assessments can be subjective, labor-intensive, and may interrupt the wound environment, potentially increasing infection risks and disrupting the healing process. Moreover, manual inspection lacks continuous data collection, making it challenging to detect subtle temperature changes that could signal infection onset.

Some recent advancements, for example as presented in US20190374387A1, have incorporated electronic components within wound dressings to automate temperature monitoring. The disclosure system uses temperature sensors embedded near the wound site to capture and transmit temperature data to an external device. The disclosed approach supports early detection of infection by analyzing temperature changes around the wound. However, the solution according to US20190374387A1 relies on threshold-based alerts, lacking the sensitivity needed for handling actual temperature patterns and baseline variations specific to each patient.

While these systems represent significant progress, there remains room for further improvement in the accuracy and specificity of temperature-based infection monitoring.

### SUMMARY

In view of the above-mentioned and other drawbacks of the prior art, it is an object of the present disclosure to provide improvements in non-invasive and accurate monitoring of infection risk at wound sites, based on acquired temperature data and relevant wound site information.

According to an aspect of the present disclosure, it is therefore provided a computer-implemented method for identifying an infection risk at a wound site, the method comprising the steps of acquiring, using a processing unit, temperature data at predefined intervals from at least one temperature sensor positioned in proximity to the wound site, processing, using the processing unit, the acquired temperature data to form a time-series of temperature values, analyzing, using the processing unit, the time-series to detect a trend in the temperature data, determining, using the processing unit, if the detected trend deviate from a predefined baseline temperature pattern associated with normal wound healing, and generating, using the processing unit, an alert signal upon detecting a deviation indicative of a potential infection risk.

The present disclosure addresses significant limitations of prior art by enabling continuous and automated monitoring of temperature trends at a wound site, using at least one but preferably a plurality of temperature sensors arranged in proximity to the wound site. Unlike methods relying on isolated temperature measurements, the present disclosure focuses on identifying patterns and deviations over time, allowing for earlier and more accurate detection of potential infection risks. By emphasizing trend-based analysis, the disclosed method reduces the likelihood of missed or delayed intervention, a common issue with traditional manual checks or single-point measurements.

The core of the present disclosure lies in leveraging advanced analytical techniques, such as regression analysis, to identify characteristic temperature trends. These include patterns such as positive or negative slopes, stepwise changes, or plateaus, which are indicative of various stages of wound healing or potential complications. Comparing these trends against a predefined baseline temperature pattern associated with normal healing enables healthcare providers to objectively evaluate the risk of infection and respond proactively.

Advantages of the disclosed method include minimizing the need for frequent physical inspection of the wound, reducing disruption to the healing process, and preserving the integrity of the wound environment. Automated detection of significant temperature deviations further supports objective assessments, reducing subjective interpretation errors and providing more reliable monitoring for healthcare providers and patients alike.

The baseline temperature pattern is preferably designed to account for natural variations in wound healing. A gradual temperature increase during the early stages of healing may be expected, while its absence could also indicate a higher risk of infection. Such a flexibility may also ensure that even complex or non-linear temperature behaviors are accurately identified, enabling timely intervention based on detailed and context-specific temperature trends.

In an embodiment, the at least one temperature sensor is positioned at a fixed predefined location relative to the wound site, or each of the temperature sensors when including a plurality of temperature sensors. By positioning the sensors in predefined and fixed locations, the present disclosure ensures consistent temperature data acquisition from multiple areas adjacent to the wound. Consistent positioning allows for the collection of reliable and comparable temperature data across different patients or monitoring sessions, which is critical for identifying trends and detecting deviations indicative of infection risk.

Fixed sensor placement may for example be achieved through integration into a wound dressing, adhesive patches, or a reusable template to guide proper application. Such arrangements ensure that temperature measurements are always taken from the same relative positions, minimizing variability caused by human error or inconsistent placement. Strategically predefined sensor locations may further allow for comprehensive coverage of the wound site, capturing data from critical regions where infection is more likely to develop, such as edges of the wound or areas of high moisture retention.

Such a configuration provides significant technical advantages, including for example consistent data collection from multiple adjacent areas that reduces noise in the dataset and improves the accuracy of temperature trend analysis. The fixed positioning also simplifies the process for clinicians, allowing for faster application and minimizing the risk of incorrect placement that could affect the reliability of the results. Ultimately, this embodiment enhances the robustness, while promoting ease of use in clinical and home care settings.

It should be noted that the phrase "acquiring, using a processing unit, temperature data at predefined intervals from a plurality of temperature sensors positioned in proximity to the wound site," as used herein, is intended to encompass various embodiments and implementations of such sensing functionality. Specifically, the term "plurality of temperature sensors" should be interpreted to include configurations where a single physical sensing unit is capable of generating temperature data from multiple discrete measurement points. For example, a single sensing unit may incorporate multiple sensing elements, or an array of micro-sensors distributed across its surface, thereby providing temperature readings from distinct locations around the wound site. Such an arrangement ensures that the required plurality of temperature measurement points is achieved without necessitating separate, physically distinct hardware units for each point, and allows for enhanced system design, reducing hardware complexity while maintaining comprehensive temperature data collection from critical regions adjacent to the wound site.

Preferably, the step of analyzing the time-series to detect a trend in the temperature data comprises performing a regression analysis to identify temperature trends over time. Regression analysis enables a precise and systematic method for evaluating temperature changes at the wound site by fitting the collected data points to a mathematical model. Such an approach allows for the possibility to identify key patterns such as positive or negative slopes, plateaus, or stepwise changes, which are indicative of normal healing or potential infection risk.

Using regression analysis enhances the ability to detect subtle deviations in the temperature data that might not be apparent through simpler analytical methods. For example, a regression-based approach can identify a gradual increase or decrease in temperature across multiple time intervals, providing early indications of infection risk even before significant temperature spikes occur. By analyzing the data trend as a whole, rather than isolated measurements, regression analysis supports a more comprehensive understanding of wound healing dynamics.

The presented exemplary embodiment offers distinct technical advantages, such as for example that it improves the accuracy and reliability of trend detection, minimizing false positives or negatives by accounting for the broader context of the temperature data. Furthermore, it provides a robust framework for automated analysis, reducing the reliance on manual interpretation and ensuring consistency across different users and scenarios. Finally, by leveraging regression techniques, it may in line with the present disclosure be possible to in an improved manner differentiate between normal healing patterns and early-stage infections, enabling timely and proactive interventions to support optimal wound care outcomes.

In some embodiments it may be possible to allow the trend detected in the temperature data to comprises at least one of a positive slope, a negative slope, a stepwise increase, or a plateau, each trend indicating a characteristic temperature change associated with predefined type of infection risk. Identifying these specific trend patterns provides a nuanced approach to monitoring wound healing and infection risk, as each trend reflects distinct physiological conditions at the wound site. For instance, a positive slope could indicate inflammation associated with normal healing or the onset of infection, while a plateau might suggest stagnation in healing progress, potentially linked to complications.

By categorizing trends into these defined patterns, it may be possible to healthcare providers to interpret temperature data in a clinically meaningful way. For example, a stepwise increase in temperature might signal an acute infection, prompting immediate intervention, whereas a gradual negative slope could reflect the resolution of inflammation and successful healing. The ability to distinguish between these patterns reduces the likelihood of false alarms and ensures that interventions are appropriately timed.

Such an approach may for example enhance the general determination, making it more reliable in both clinical and home care settings. Additionally, the predefined patterns provide a clear framework for automating infection risk assessment, reducing the burden on healthcare providers to interpret raw data manually. Accordingly, the scheme according to the present disclosure may be used for not only identifying deviations but also contextualizing them in terms of their clinical significance, thereby supporting more informed and effective wound care management.

In accordance with the present disclosure, it is made possible to incorporate historical data to provide an individualized approach to monitoring, making it possible to recognize patterns and deviations unique to the healing trajectory for each wound. Accordingly, the method according to the present disclosure may adjust its analysis to better reflect the individual characteristics of the healing process. For instance, a wound exhibiting naturally higher baseline temperatures due to its location, or the physiological state of the patient can be monitored more accurately, as the historical data provides the necessary context. Deviations from this personalized trend can then signal potential complications, such as infection or delayed healing, even when these changes might be too subtle to detect using general baseline patterns.

Tailoring the analysis to specific historical temperature trends for the wound improves the precision of infection risk detection by reducing false positives and negatives that may arise from generalized assumptions. The use of historical data also allows for more effective longitudinal monitoring, providing healthcare providers with insights into the progression of the wound over time and its response to treatment. Moreover, such an approach strengthens early detection capabilities, ensuring that even minor deviations from the expected healing pattern are identified in time to enable proactive intervention.

In an embodiment, the predefined baseline temperature pattern comprises a range of acceptable temperature values and expected temperature trends associated with normal wound healing over a predefined period. Defining the baseline in such a way provides a structured and adaptable framework for comparing temperature data, allowing for the possibility to account for variations in normal healing patterns while maintaining sensitivity to deviations indicative of infection risks.

The range of acceptable temperature values and expected trends may be derived from clinical studies, patient-specific factors, or population-based norms. For example, normal healing may exhibit a gradual increase in temperature over the first few days followed by stabilization, and the baseline can reflect these dynamics over a predefined monitoring period. Such a range ensures flexibility, accommodating physiological differences between patients and wound types while remaining focused on deviations that signal potential complications.

The use of both temperature ranges and expected trends may also ensure that the determination is not overly rigid, reducing the risk of false alerts caused by minor, clinically irrelevant fluctuations. Furthermore, the predefined period provides context for interpreting changes, ensuring that deviations are assessed within an appropriate time frame.

Preferably, the step of generating an alert signal comprises transmitting the alert signal to a remote device configured to notify a healthcare provider or a patient of the potential infection risk. Enabling remote transmission ensures that critical information about the condition of the wound is communicated promptly, regardless of the location of the recipient.

The alert signal may convey relevant details, such as the type of detected deviation, its magnitude, and the time it was identified. Notifications can be sent to various devices, such as a monitoring system used by the healthcare provider, a smartphone used by the patient, or a device used by a caregiver. For example, clinicians could receive alerts directly on a hospital dashboard, while patients may receive notifications via a dedicated mobile application, allowing them to take action swiftly.

Furthermore, providing remote notifications ensures rapid response times and improved accessibility to critical data. Healthcare providers benefit from detailed alerts, enabling them to assess the situation and determine appropriate interventions without delay. Patients are empowered to actively monitor the progress of their healing and take necessary steps as recommended by their healthcare team. Additionally, integrating remote notifications into telemedicine platforms makes this solution highly adaptable for home care settings and regions with limited access to in-person medical services.

Still further, in some embodiments it may be possible to allow the plurality of temperature sensors to be combined with a wound dressing positioned at the wound site or provided as a monitoring arrangement in vicinity of the wound site. Combining the sensors with a wound dressing ensures seamless integration, allowing for continuous temperature monitoring while minimizing disruption to the wound during routine care.

The temperature sensors can be embedded directly within the material of the wound dressing or adhered to its surface, ensuring they remain in proximity to the wound site throughout the healing process. Alternatively, a dedicated monitoring arrangement positioned near the wound site can offer flexibility for applications where sensor integration into the dressing is not feasible. These configurations allow temperature data to be captured consistently, even during dressing changes or when the wound is being treated.

Integrating temperature sensors with a wound dressing or using a nearby monitoring arrangement provides distinct advantages, namely for example by ensuring that subtle temperature trends are detected early, enabling timely intervention without the need for frequent manual checks. This approach preserves the integrity of the wound by reducing unnecessary handling, which minimizes the risk of infection and supports optimal healing conditions. Additionally, such arrangements enhance patient comfort by allowing data to be collected passively, eliminating the need for invasive procedures or repeated sensor repositioning.

Furthermore, in some embodiments the scheme according to the present disclosure may comprise the step of updating the predefined baseline temperature pattern over time based on a historical time-series of temperature values previously collected at the wound site. Dynamic adjustment of the baseline allows the monitoring process to account for patient-specific healing profiles or incorporate insights derived from broader clinical knowledge.

By comparing ongoing temperature data with a predefined time-series, the baseline can be refined to reflect individual variations in healing processes. Unique physiological responses or external factors affecting a specific patient may lead to deviations from generalized healing patterns. Regular updates ensure that such variations are accurately captured, reducing the likelihood of false alarms or missed infection risks. Adjustments can also be informed by data from clinical studies or accumulated knowledge, enhancing the relevance of the baseline to a wide range of cases.

Such a capability may also enable precise detection of significant deviations while accommodating individual differences and evidence-based insights. Providing patient-specific or knowledge-driven adjustments ensures a flexible and robust monitoring framework, supporting reliable infection risk assessments and improving outcomes for diverse patient populations and wound types.

In an embodiment, the scheme according to the present disclosure further comprises the step of generating a visual representation of the detected temperature trends and deviations, the visual representation being configured to assist a healthcare provider in assessing the infection risk at the wound site. Providing a clear and interpretable visualization of temperature data enhances the ability of healthcare providers to make informed decisions based on the analysis being performed in line with the present disclosure.

The visual representation may include graphical elements such as line graphs to illustrate temperature trends over time, markers to highlight deviations from the baseline, or color-coded indicators to emphasize areas of concern. Such visualizations allow the healthcare provider to quickly interpret the data and identify patterns that may indicate infection risk. For example, a trend showing a rapid increase in temperature could be visually highlighted, prompting immediate attention, while stable trends within acceptable ranges may be displayed in a manner that signals normal healing progress.

Such an embodiment may facilitate a more intuitive understanding of complex temperature data, making it accessible to providers without requiring in-depth technical expertise. By presenting the information in a clear and concise format, the visualization reduces the cognitive load on healthcare professionals, allowing them to focus on decision-making and patient care. The inclusion of visual representations also supports better communication with patients, enabling them to understand the healing progress and engage more actively in their care.

Preferably, the time-series of temperature values is stored in a patient monitoring system for longitudinal analysis of temperature trends across one or multiple wound sites on the same patient. Storing the data enables tracking of temperature patterns over an extended period, offering a valuable resource for assessing individual wound healing progress and the overall health status of the patient.

In the embodiment where multiple wound sites, it may accordingly be possible to adapt the patient monitoring system to organize temperature data by each wound site, allowing healthcare providers to view and compare trends for multiple wounds concurrently. Deviations in temperature trends across several wounds could indicate systemic health issues or a more localized infection. Similarly, storing and analyzing trends over time provides insights into the effectiveness of specific treatments or interventions, allowing care plans to be adjusted based on data-driven observations.

Longitudinal storage of temperature data improves the accuracy of infection detection and supports more informed decision-making in wound care management. The availability of historical data ensures continuity of care, enabling healthcare providers to access a comprehensive record of the patient's condition and collaborate effectively. Additionally, the stored data allows for retrospective reviews, which can contribute to improved wound care practices and better outcomes for future patients.

In addition to the above, in some embodiments it may be possible to allow the scheme according to the present disclosure to further comprise the step of comparing the detected trend in the temperature data to a set of predefined temperature patterns, each pattern associated with a specific infection risk profile, to identify a potential type of infection risk. Adding such a comparison enables for the possibility to provide detailed insights into the nature of the infection risk, moving beyond merely signaling the presence of a deviation.

The predefined temperature patterns may include trends such as rapid increases, prolonged plateaus, or fluctuating values, each correlated with specific conditions like bacterial infection, wound stagnation, or inflammation. By matching the detected trend against these predefined profiles, it may in line with the present disclosure be possible to identify likely causes of the temperature deviations. For example, a rapid increase in temperature could indicate a bacterial infection, while a stable plateau might point to delayed healing or insufficient vascular response.

Incorporating the ability to classify infection risks allows healthcare providers to implement more precise and effective interventions. Recognizing the type of infection risk ensures that care plans can be adjusted accordingly, whether by modifying wound care procedures or initiating targeted treatments. Furthermore, the ability to categorize risks enhances collaboration among care teams by providing detailed and actionable information.

Additionally, in some embodiments it may be possible to arrange the predefined intervals for acquiring temperature data to be selected based on a real-time monitoring requirement. Configuring the data acquisition intervals in this manner ensures flexibility in adapting the monitoring process to the needs of the specific wound or clinical situation, allowing for timely detection of changes in temperature that may signal the onset of an infection.

Shorter intervals between temperature measurements can be selected when real-time or near-real-time monitoring is necessary, such as in cases where the patient is at high risk of infection or during the critical stages of wound healing. Such an approach may possibly enable the detection of rapid temperature deviations that may indicate sudden infection onset, ensuring prompt alerts and immediate intervention. Conversely, longer intervals may be chosen for stable wounds or during periods when less frequent monitoring is sufficient.

The ability to adapt the data acquisition intervals to the monitoring requirements generally may be seen as improving the responsiveness and efficiency. Real-time monitoring enhances the detection of rapid temperature changes, enabling healthcare providers to act quickly and reduce the risk of complications. Additionally, the flexibility in selecting intervals optimizes the use of resources by focusing monitoring efforts where they are most needed, without compromising the accuracy or reliability of the collected data.

In some embodiments is may be suitable to further include a step of applying a filter to the detected trend to reduce false-positive alerts. Implementing a filtering mechanism minimizes the impact of minor fluctuations or noise in the temperature data, ensuring that only significant deviations are considered, thereby improving the accuracy and reliability of the monitoring process.

Filters may be configured as trend filters, threshold filters, band-pass filters, high-pass filters, or low-pass filters, depending on the specific requirements of the application. A threshold filter can exclude minor deviations below a predefined magnitude, focusing on significant changes in temperature that may indicate infection risks. A band-pass filter can target temperature changes within a specific range most relevant to normal healing or infection onset, while a high-pass filter can eliminate gradual baseline shifts that are less likely to represent a clinical concern.

Applying a filter ensures that false-positive alerts are reduced, allowing healthcare providers to prioritize genuine risks. Minimizing unnecessary alerts avoids overburdening clinicians with irrelevant information and reduces unnecessary interventions, promoting greater treatment efficiency and patient comfort. Filtering also makes it possible to tailor the monitoring process to specific wound types or patient profiles, ensuring the monitoring process remains adaptable and precise in diverse clinical scenarios.

According to another aspect of the present disclosure, there is provided a computer system for identifying an infection risk at a wound site, wherein the computer system comprises a processing unit adapted to acquire temperature data at predefined intervals from at least one temperature sensor positioned in proximity to the wound site, process the acquired temperature data to form a time-series of temperature values, analyze the time-series to detect a trend in the temperature data, determine if the detected trend deviate from a predefined baseline temperature pattern associated with normal wound healing, and generate an alert signal upon detecting a deviation indicative of a potential infection risk. This aspect of the present disclosure provides similar advantages as discussed above in relation to the previous aspects of the present disclosure.

In a possible embodiment of the present disclosure, the computer system is a handheld electronic device, further comprising a display screen for presenting a graphical user interface (GUI) and an interface for forming a connection with the temperature sensors. The handheld nature of the device ensures portability and accessibility, enabling both healthcare providers and patients to utilize the system conveniently in various care settings.

The GUI presented on the display screen could be designed to facilitate not only temperature monitoring but also a combined assessment of infection risk. For example, the GUI could prompt regular questions to the patient, tailored to trends detected in the temperature data. If a temperature trend indicates a potential risk, the system could ask the patient about additional symptoms, such as the presence of pain, swelling, discharge, exudate or redness in relation to the wound site(s), and/or the patient feeling e.g. fever or nausea. The GUI may also enable visual inspection, allowing patients or caregivers to capture images of the wound, which can be integrated into the assessment process. Accordingly, in some embodiments the processing unit is adapted to acquire an indication of a perceived symptom, which then is taken into account when generating the alert signal, in the manner as discussed above.

Such a combined assessment approach enhances the accuracy and depth of infection risk evaluations. By considering multiple factors, such as pain levels, visible swelling, and temperature trends, the system provides a more comprehensive understanding of the condition of the wound. Additionally, the handheld portability of the handheld device and user-friendly interface allow for seamless communication between patients and healthcare providers, enabling timely responses to emerging risks. Furthermore, the inclusion of a GUI simplifies the interaction, ensuring that both professionals and non-experts can engage with the system effectively.

Preferably, the processing unit is further adapted to generate an alert when the detected trend in temperature data exceeds a predefined infection risk threshold, and to display the alert within the GUI on the display screen. By integrating the alert system directly into the GUI, the computer system ensures that critical information is conveyed in an accessible and actionable manner, enabling users to respond promptly to potential risks.

The alert may include details such as the type and severity of the detected deviation, a visual representation of the temperature trend, and specific recommendations for next steps, such as consulting a healthcare provider or inspecting the wound. The GUI can be designed to prioritize clarity, using visual elements like color-coded warnings or flashing indicators to ensure the alert is immediately noticeable.

Presenting the alert within the GUI on the display screen enhances the usability of the system. Healthcare providers gain immediate insights into the status of the wound, allowing for faster clinical decisions, while patients are empowered to take proactive steps to address potential complications. Integrating alerts into the GUI ensures that important information is centralized and presented in a format that is easy to interpret, reducing the likelihood of miscommunication or delays in responding to infection risks.

In some embodiments the computer system as discussed above is provided as a component of a wound treatment system, further comprising a wound dressing, and a measurement arrangement provided in a vicinity of the wound site. Integrating the computer system into a wound treatment system ensures seamless interaction between temperature monitoring, wound care, and infection risk assessment, creating a comprehensive solution for managing wound healing.

The wound dressing may incorporate temperature sensors strategically positioned to capture data from key areas near the wound site. The measurement arrangement, which can include additional monitoring tools, ensures consistent data collection without disrupting the wound environment. The computer system processes the data captured by the sensors and provides actionable insights via its integrated analysis and alert mechanisms.

Providing the computer system as part of a wound treatment system enhances its functionality and clinical value. Combining temperature monitoring with a physical dressing ensures continuous data acquisition while maintaining optimal conditions for healing. The inclusion of a measurement arrangement allows for flexibility in how data is collected and transmitted, accommodating various clinical and home care settings.

According to a further aspect of the present disclosure, there is provided a computer program comprising instructions which, when executed by a computer or processor, cause a computer system to identify an infection risk at a wound site, wherein the computer system comprises a processing unit, and wherein the computer program comprises code for acquiring, using a processing unit, temperature data at predefined intervals from at least one temperature sensor positioned in proximity to the wound site, code for processing, using the processing unit, the acquired temperature data to form a time-series of temperature values, code for analyzing, using the processing unit, the time-series to detect a trend in the temperature data, code for determining, using the processing unit, if the detected trend deviate from a predefined baseline temperature pattern associated with normal wound healing, and code for generating, using the processing unit, an alert signal upon detecting a deviation indicative of a potential infection risk. Also this aspect of the present disclosure provides similar advantages as discussed above in relation to the previous aspects of the present disclosure.

A software executed by the processing unit for operation in accordance to the present disclosure may be stored on a computer readable medium, being any type of memory device, including one of a removable non-volatile random access memory, a hard disk drive, a floppy disk, a CD-ROM, a DVD-ROM, a USB memory, an SD memory card, a solid state drive, other non-volatile flash based storage media, or a similar computer readable medium known in the art.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Fig. 1 conceptually illustrates a computer system according to a currently preferred embodiment of the present disclosure,
Figs. 2A and 2B present exemplary temperature graphs illustrating temperature variations in a vicinity of a wound of a patient, and
Fig. 3 is a flow chart illustrating the steps of performing the method according to a currently preferred embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

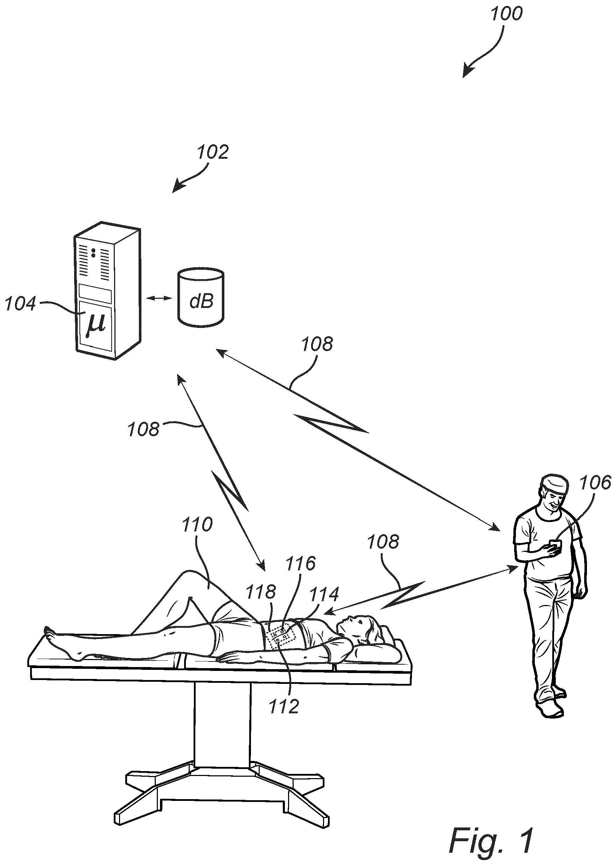

Turning now to the drawings and to Fig. 1 in particular, there is conceptually illustrated a computer system 100 configured for identifying infection risk at a wound site. The computer system 100 comprises interconnected components designed to acquire temperature data from a patient 110, analyze the data, and provide actionable alerts based on the analysis. The system is specifically designed for non-invasive, real-time monitoring of wound conditions, supporting early identification of infection risks.

The computer system 100 includes a server 102, which acts as the central hub for processing and storing temperature data. The server 102 is configured to manage data acquisition, analysis, and communication between various components. A processing unit 104 is, for example, integrated within the server 102 and is responsible for executing analytical algorithms. These algorithms include, but are not limited to, the steps of forming time-series data, trend detection, and comparing detected trends to predefined baseline patterns associated with normal wound healing.

For reference, the processing unit 104 may for example be manifested as a general-purpose processor, a graphics processing unit, an application specific processor, a circuit containing processing components, a group of distributed processing components, a group of distributed computers configured for processing, a field programmable gate array (FPGA), etc. The processor may be or include any number of hardware components for conducting data, signal and/or image processing or for executing computer code stored in memory. It may also be possible and within the scope to make use of system-on-chip (SOC) implementations. The memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to an exemplary embodiment, any distributed or local memory device may be utilized with the systems and methods of this description. According to an exemplary embodiment the memory is communicably connected to the processor (e.g., via a circuit or any other wired, wireless, or network connection) and includes computer code for executing one or more processes described herein.

The server 102 is in Fig. 1 illustrated as connected to a handheld unit 106 via a network connection 108. The network connection 108 may include a wired connection, such as Ethernet, or a wireless connection, such as Wi-Fi or Bluetooth, to facilitate secure and reliable data transmission. The handheld unit 106 serves as a user interface for healthcare providers and/or patients and includes a display screen. The display screen is configured to present a graphical user interface (GUI), which provides access to data visualizations, alerts, and control features. Through the GUI, healthcare providers or patients can view real-time temperature trends, analyze potential deviations, and receive actionable recommendations. The handheld unit 106 may for example include a mobile phone.

A patient 110 is equipped with a measurement arrangement 112, in the exemplified embodiment comprising a plurality of temperature sensors 116. The measurement arrangement 112 is configured to collect temperature data from multiple locations in a vicinity of a wound site 114. The temperature sensors 116 are strategically arranged in a predefined spatial pattern to ensure uniform and consistent data collection across critical regions at the wound site 114. The predefined pattern is designed to capture temperature variations in areas most likely to exhibit early signs of infection, such as wound edges or regions prone to moisture accumulation. Within the context of the present disclosure, it may in some alternative embodiments be possible to use only a single temperature sensor 116.

The temperature sensors 116 may alternatively (or also) be embedded in a wound dressing 118, adhered to the skin of the patient using adhesive pads at wound site 114. Preferably, each temperature sensor 116 is calibrated to ensure accurate and reliable measurements, even under varying environmental conditions. In a typical configuration, the measurement arrangement 112 may include four temperature sensors 116, although more or fewer sensors may be used depending on the wound's size or the monitoring requirements. The use of multiple sensors ensures comprehensive coverage of the wound site, capturing thermal variations across different regions.

The data collected from the sensors 116 is averaged to provide a mean temperature value, which offers a concise representation of the overall thermal behavior of the wound site. Such an averaging process also enhances the reliability of the data by mitigating the influence of localized anomalies or sensor-specific deviations. Furthermore, outliers caused by temporary disturbances, such as patient movement or environmental changes, may be identified and removed using filtering algorithms to ensure the integrity of the dataset. The sensors 116 collect temperature data at regular predefined intervals, which are set based on real-time monitoring requirements or clinical guidelines for wound care.

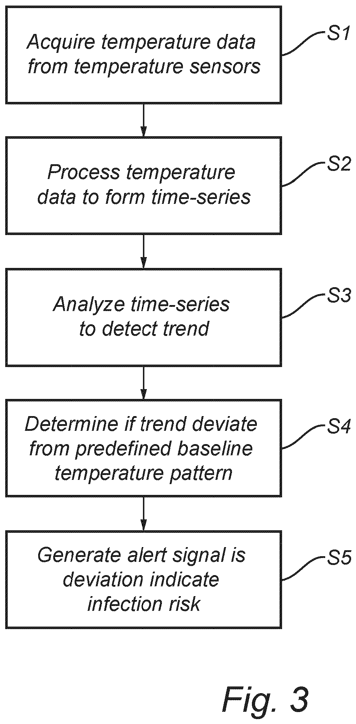

The measurement arrangement 112 (or alternatively the wound dressing provided with the sensors 116) transmits the collected temperature data to the server 102 via a communication interface, such as the network connection 108. Such an interface may include wireless protocols such as Bluetooth Low Energy (BLE), Zigbee, or Wi-Fi, or alternatively, a wired connection for scenarios requiring enhanced data security or stability. The transmitted data is received and processed by the server 102, where the processing unit 104 executes computational steps to identify potential infection risks. These steps, as will be detailed further in relation to Fig. 3, include forming time-series data, detecting trends using regression analysis, and comparing the detected trends to baseline patterns to determine deviations indicative of infection risks.

It is worth noting that the processing unit 104 in some alternative embodiments may be comprised with the handheld device 106, whereby the handheld device 106 received the measurement data from the measurement arrangement 112 and performs the processing according to the present disclosure. Possibly, the results may subsequently be transmitted to the server 102 for storage.

The network connection 108 enables bi-directional communication between the server 102 and the handheld unit 106, wherein the connection is provided for ensuring that alerts or visual representations of temperature trends generated by the processing unit 104 are transmitted to the handheld unit 106 in real-time. These alerts can include details about the type of detected deviation, its severity, and recommendations for further action. For example, an alert may notify a healthcare provider to inspect the wound or initiate a targeted treatment, while providing visualizations such as line graphs or heatmaps to illustrate temperature trends.

The measurement arrangement 112 is preferably designed to operate in a non-invasive manner. By collecting data continuously and passively, it avoids the need for frequent manual inspections or disturbance of the wound environment. Such an implementation preserves the integrity of the wound, minimizes the risk of secondary infections, and supports optimal healing conditions. Additionally, the non-invasive design prioritizes patient comfort and convenience, particularly during extended periods of wound monitoring. By eliminating the need for repeated manual inspections or invasive procedures, the system reduces physical discomfort and alleviates psychological stress often associated with frequent clinical interventions. Furthermore, the predefined configuration of the temperature sensors 116 ensures consistent and reproducible data collection, which is critical for identifying subtle trends and deviations that may indicate early signs of infection. Together, these features enhance the usability and acceptance of the system for both clinical and home care applications, enabling more effective and patient-centered wound management.

During operation of the computer system 100, and with further reference to Fig. 3, the method for identifying an infection risk at a wound site is initiated by acquiring, S 1, temperature data from the measurement arrangement 112. The sensors 116 are configured to collect temperature readings at predefined intervals, ensuring continuous monitoring of the wound site 114, and the collected temperature data is transmitted to the server 102 either wirelessly, using communication protocols such as Bluetooth, Wi-Fi, or Zigbee, or via a wired connection utilizing standardized interfaces.

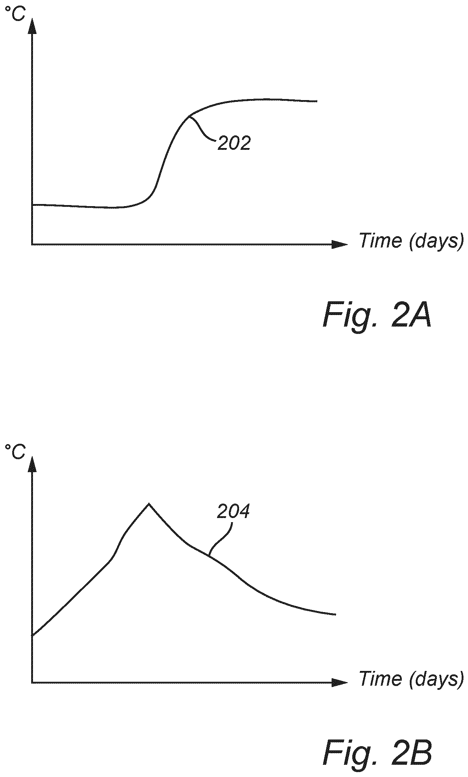

The acquired temperature data is subsequently processed, S2, by the processing unit 104. As an example, the raw temperature readings may be organized into a structured time-series format, where each data point is associated with a precise timestamp. Such an organization facilitates the detection of temporal trends in the thermal activity at the wound site. Additionally, the system 100 may apply noise reduction techniques to filter out anomalous readings, such as transient spikes caused by sensor miscalibration, ensuring that the dataset is accurate and reliable. The processed data is then consolidated into a mean temperature graph 202, as shown in Fig. 2A, here illustrating the average temperature values derived from the plurality of sensors 116 over time, providing a clear representation of the overall thermal behavior of the wound site. The x-axis of the graph represents time (e.g., days), while the y-axis represents the mean temperature (e.g., degrees Celsius).

Once the data is processed, the processing unit 104 analyzes, S3, the time-series to detect trends in the temperature readings. The processing unit 104 may for example apply regression analysis to the time-series, enabling the detection of significant patterns such as positive or negative slopes, stepwise changes, or sustained plateaus. These trends are indicative of different physiological conditions at the wound site.

The use of regression analysis for trend detection provides a significant advantage over simpler methods, such as threshold-based monitoring, by capturing the overall behavior of temperature changes over time. While threshold methods might flag isolated temperature spikes as potential risks, regression analysis considers the broader context, distinguishing between transient fluctuations and meaningful trends. Accordingly, such an approach minimizes false-positive alerts that could otherwise lead to unnecessary interventions and reduces false negatives by identifying gradual or subtle deviations that simpler methods might overlook.

Following the trend analysis, the system proceeds to determine, S4, whether the detected trend deviates from a predefined baseline temperature pattern stored in the server 102. This baseline represents the physiologically acceptable range of temperature values and trends associated with normal wound healing. For example, a typical baseline might include an initial rise in temperature due to the natural inflammatory response, followed by stabilization as the wound heals.

That said, baseline patterns may in some embodiments of the present disclosure be tailored to specific wound types (e.g., surgical incisions, pressure ulcers) or adjusted based on patient-specific factors such as age, comorbidities, or healing rates. Such individualization may further enhance the accuracy of deviation detection.

Furthermore, it is important to note that the trend detection step is not necessarily independently decisive in determining the infection risk. The detected trend should rather preferably be evaluated in relation to the predefined baseline pattern to ensure a reliable assessment. For instance, while a positive slope might initially indicate a potential infection risk, such a trend could still fall within the baseline if it corresponds to a known phase of normal wound healing, such as the early inflammatory response. Conversely, deviations that extend beyond the predefined range of the baseline are more likely to signal complications requiring attention.

However, in Fig. 2A, the mean temperature graph 202 exhibits a sustained increase beyond the acceptable range (e.g., exceeding 42°C), which suggests the presence of abnormal activity, such as bacterial infection or delayed healing. Specifically, the trend analysis in conjunction with the comparison identifies a sustained and sharp increase in the mean temperature over time, deviating significantly from the expected trend for normal wound healing. Such a positive slope may as such be flagged as a potential indicator of infection risk.

Based on the evaluation, the processing unit 104 generates, S5, an alert signal indicating the identified risk. The alert is transmitted to the handheld unit 106 via the network connection 108. On the graphical user interface (GUI) of the handheld unit, the mean temperature graph 202 is displayed with annotations highlighting the problematic trend and deviation from the baseline. Such an alert equips healthcare providers or patients with actionable information to facilitate timely intervention. In addition, the GUI may provide options for further actions, such as reviewing historical data or contacting a healthcare provider directly.

Additionally, in Fig. 2B there is provided an additional illustrative example of a further temperature graph 204. Conversely to the illustration as presented in Fig. 2A, the graph 204 as shown in Fig. 2B represents a situation where the thermal behavior of the wound follows an expected pattern, and no deviations indicative of infection risk are detected. Again, temperature data is acquired at regular intervals from the sensors 116, ensuring consistent and reliable monitoring of the wound site. These readings form a dataset, and the mean temperature graph 204 is derived.

As seen in Fig. 2B, the graph 204 depicts an initial mild temperature increase, followed by a gradual decrease as the wound progresses toward recovery. The processing unit 104 subsequently analyzes the detected trend using regression techniques to identify meaningful patterns in the data. In this case, the steady downward slope of the graph indicates a stable progression of healing without any signs of complications. The detected trend is compared to the predefined baseline pattern as discussed above, confirming that it falls within the acceptable range of values and expected trajectories for normal healing.

Since the thermal behavior observed in Fig. 2B aligns with the baseline, no alert is generated. Instead, the computer system 100 may transmit the processed data and results to the handheld unit 106. The graphical user interface (GUI) on the handheld device may accordingly display the mean temperature graph 204, along with annotations confirming the absence of infection risk and notifying the user that the wound is healing as expected. However, even though no risk for invention is identified, the GUI may provide positive reinforcement by displaying annotations indicating normal healing progress, reassures patients and supports adherence to prescribed wound care protocols.

Furthermore, the control functionality of the present disclosure may be implemented using existing computer processors, or by a special purpose computer processor for an appropriate system, incorporated for this or another purpose, or by a hardwire system. Embodiments within the scope of the present disclosure include program products comprising machine-readable medium for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, solid state drives or other non-volatile flash based storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions.

Although the figures may show a sequence the order of the steps may differ from what is depicted. Also two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps. Additionally, even though the present disclosure has been described with reference to specific exemplifying embodiments thereof, many different alterations, modifications and the like will become apparent for those skilled in the art.

In addition, variations to the disclosed embodiments can be understood and effected by the skilled addressee in practicing the claimed present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A computer-implemented method for identifying an infection risk at a wound site, the method comprising the steps of:
acquiring, using a processing unit, temperature data at predefined intervals from at least one temperature sensor positioned in proximity to the wound site,
processing, using the processing unit, the acquired temperature data to form a time-series of temperature values,
analyzing, using the processing unit, the time-series to detect a trend in the temperature data,
determining, using the processing unit, if the detected trend deviate from a predefined baseline temperature pattern associated with normal wound healing, and
generating, using the processing unit, an alert signal upon detecting a deviation indicative of a potential infection risk.

2. The method according to claim 1, wherein the at least one temperature sensor is positioned at a fixed predefined location relative to the wound site.

3. The method according to any one of claims 1 and 2, wherein the step of analyzing the time-series to detect a trend in the temperature data comprises performing a regression analysis to identify temperature trends over time.

4. The method according to any one of the preceding claims, wherein the trend detected in the temperature data comprises at least one of a positive slope, a negative slope, a stepwise increase, or a plateau, each trend indicating a characteristic temperature change associated with predefined type of infection risk.

5. The method according to any one of the preceding claims, wherein the predefined baseline temperature pattern comprises a range of acceptable temperature values and expected temperature trends associated with normal wound healing over a predefined period.

6. The method according to any one of the preceding claims, wherein the step of generating an alert signal comprises transmitting the alert signal to a remote device configured to notify a healthcare provider or a patient of the potential infection risk.

7. The method according to any one of the preceding claims, wherein the at least one temperature sensor is associated with a wound dressing positioned at the wound site, or provided as a separate monitoring arrangement to be positioned in a vicinity of the wound site.

8. The method according to any one of the preceding claims, further comprising the step of updating the predefined baseline temperature pattern over time based on a historical time-series of temperature values previously collected at the wound site.

9. The method according to any one of the preceding claims, wherein the time-series of temperature values is stored in a patient monitoring system for longitudinal analysis of temperature trends across one or multiple wound sites on the same patient.

10. The method according to any one of the preceding claims, further comprising a step of applying a filter to the detected trend to reduce false-positive alerts.

11. The method according to any one of the preceding claims, further comprising the step of:
acquiring an indication of a perceived symptom, wherein the perceived symptom is taken into account by the processing unit when generating the alert signal.

12. A computer system for identifying an infection risk at a wound site, wherein the computer system comprises a processing unit adapted to:
acquire temperature data at predefined intervals from at least one temperature sensor positioned in proximity to the wound site,
process the acquired temperature data to form a time-series of temperature values,
analyze the time-series to detect a trend in the temperature data,
determine if the detected trend deviate from a predefined baseline temperature pattern associated with normal wound healing, and
generate an alert signal upon detecting a deviation indicative of a potential infection risk.

13. The computer system according to claim 15, wherein the computer system is a handheld electronic device, further comprising a display screen for presenting a graphical user interface (GUI) and an interface for forming a connection with the at least one temperature sensor.

14. A wound treatment system, comprising:
a computer system according to any one of claims 12 - 13, and
a wound dressing, and
a plurality of temperature sensors provided in vicinity of a wound site.

15. A computer program comprising instructions which, when executed by a computer or processor, cause a computer system to identify an infection risk at a wound site, wherein the computer system comprises a processing unit, and wherein the computer program comprises:
code for acquiring, using a processing unit, temperature data at predefined intervals from at least one temperature sensor positioned in proximity to the wound site,
code for processing, using the processing unit, the acquired temperature data to form a time-series of temperature values,
code for analyzing, using the processing unit, the time-series to detect a trend in the temperature data,
code for determining, using the processing unit, if the detected trend deviate from a predefined baseline temperature pattern associated with normal wound healing, and
code for generating, using the processing unit, an alert signal upon detecting a deviation indicative of a potential infection risk.
